Europäisches Patentamt

European Patent Office

Office européen des brevets

(11) Publication number: **0 209 246**
A1

# (12) EUROPEAN PATENT APPLICATION

(21) Application number: 86304529.0

(22) Date of filing: 12.06.86

(51) Int. Cl.⁴: **A 61 N 1/42**, B 60 N 1/00

(30) Priority: 12.06.85 AU 1004/85

(43) Date of publication of application: 21.01.87
Bulletin 87/4

(84) Designated Contracting States: DE FR GB IT

(71) Applicant: **Daw, Graeme Ernest, 28 Hampshire Road, Glen Waverley Victoria 3150 (AU)**

(72) Inventor: **Daw, Graeme Ernest, 28 Hampshire Road, Glen Waverley Victoria 3150 (AU)**

(74) Representative: **Frost, Dennis Thomas et al, WITHERS & ROGERS 4 Dyer's Buildings Holborn, London, EC1N 2JT (GB)**

(54) **Magnetic therapy device.**

(57) A magnetic therapy device comprising a pad (10) which is adapted to be in close contact with a user when in use, the pad including at least one elongated magnetic coil (11, 12) which is/are adapted to be located adjacent the portion of the body to be treated, a support (13) upon which the coil(s) are located and a covering (15) for the coil. Also, there is an associated excitation means to produce a magnetic field in the coil(s) of the device which includes a pulse generator and a power amplifier.

# MAGNETIC THERAPY DEVICE

This invention relates to a magnetic therapy device and, in particular, to a magnetic therapy device which is particularly useful in such applications as to relax the muscles of and remove pain from persons who spend long periods driving vehicles.

The invention is by no means restricted to this application but I shall use this as an example.

Magnetic therapy has passed from an area subject to skepticism to being basically well accepted and two areas in which it is most used are in treatment and, specifically, healing of breaks and the treatment of infection and in the area of muscle relaxation, the minimising of bruising and pain removal.

In each of these areas it has been usual to locate solid magnetic coils adjacent or on each side of the area to be treated and to provide these coils with current at between 1 and 200 Hz and of a value such as to induce a magnetic field of the order of 100 gauss. However combinations of all frequencies and gauss have proved an effective treatment.

In the second form of application, where used as a muscle relaxant for the minimisation of bruising and for pain removal, coils of a similar type are used and, in this case, it is not unusual to use a frequency at the lower end of the range, of the order of 5 Hz and a field of a lower order, of the order of 15 gauss.

Whilst the results obtained by such treatment can be quite astonishing, it has, nevertheless, been inconvenient to provide the coils and to locate these relative to a user as the coils are usually relatively large and clumsy to handle

The object of the present invention is to provide a magnetic therapy device whereby a magnetic field can be easily applied to a user and, preferably, can be left in a position so that the field can be maintained whilst the user carries out his or her normal functions.

Specifically, the invention provides such a device for use by drivers who are in vehicles for long periods.

The invention, in its broadest sense, comprises a magnetic therapy device being a flexible pad which is adapted to be in close contact with a user when in use, which pad includes at least one elongated magnetic coil which is adapted to be located adjacent the portion of the body of the user to be treated, a support upon which this coil is located and a covering for the coil.

Preferably the coil may be in a sandwich of a material having no magnetic properties, such as a flexible foam material and there may be an external cover which has a surface against which it is pleasant to rest, for example lambswool.

The field in the magnetic coil may be induced by an electronic device having an on/off radio of 1:4 operating, say, on a 20 millisecond cycle with the cycle being effected for 100 milliseconds and broken for the next 100 milliseconds, that is a 5 Hz signal. Preferably there may be more than one coil and the coils may be located side by side and be of different polarity.

In order that the invention may be more readily understood, I shall describe, in relation to the accompanying drawings, one particular form of device made in accordance with the invention, which form is adapted for use in a vehicle.

In these drawings:

Fig. 1    illustrates schematically the pad of the invention as fitted to a vehicle seat; and

Fig. 2    is a block diagram showing an arrangement whereby the pad can be activated.

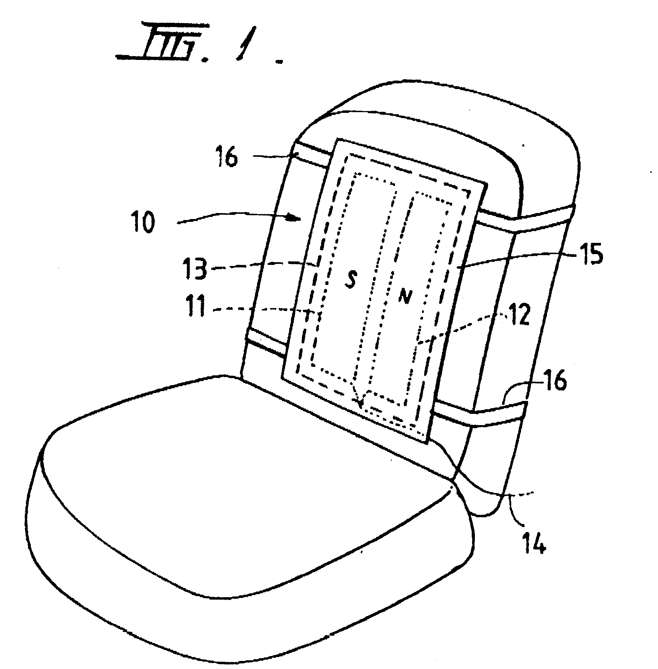

In the flexible pad 10, illustrated in Fig. 1, I may have two coils 11, 12, each of which may comprise thirty turns of wire and each of which is in the form of a rectangle having a longer side of, say, 750 mm and a width of, say, 150 mm with the two coils being adjacent with their 750 mm sides contiguous.

One coil is preferably wound in such a way as to be effectively a north coil, and this is so indicated on the Figure, and the other, in the opposite way, to be effectively a south coil.

0209246

These coils are preferably located as the centre of a sandwich made of two layers of a flexible synthetic plastics foam, shown generally as 13, which may form a surround of a size slightly greater than the area occupied by the coils, say 800 to 850 mm long by 350 to 500 mm wide.

Extending from the sandwich are the electrical wires 14 necessary to cause current to flow through the coils and to induce magnetism thereby.

The sandwich may be covered with a wearing material, shown generally as 15, and in one preferred form, may be covered with a lambswool cover which provides a comfortable surface against which the user can rest.

The pad is, as illustrated, provided with tapes or elastic straps 16 or the like to enable it to be attached to a seat back or other surface. As illustrated in Fig. 1, the pad is shown attached to a vehicle seat.

Alternatively, the pad could be provided with means whereby it can be retained after being wrapped about an area to be treated.

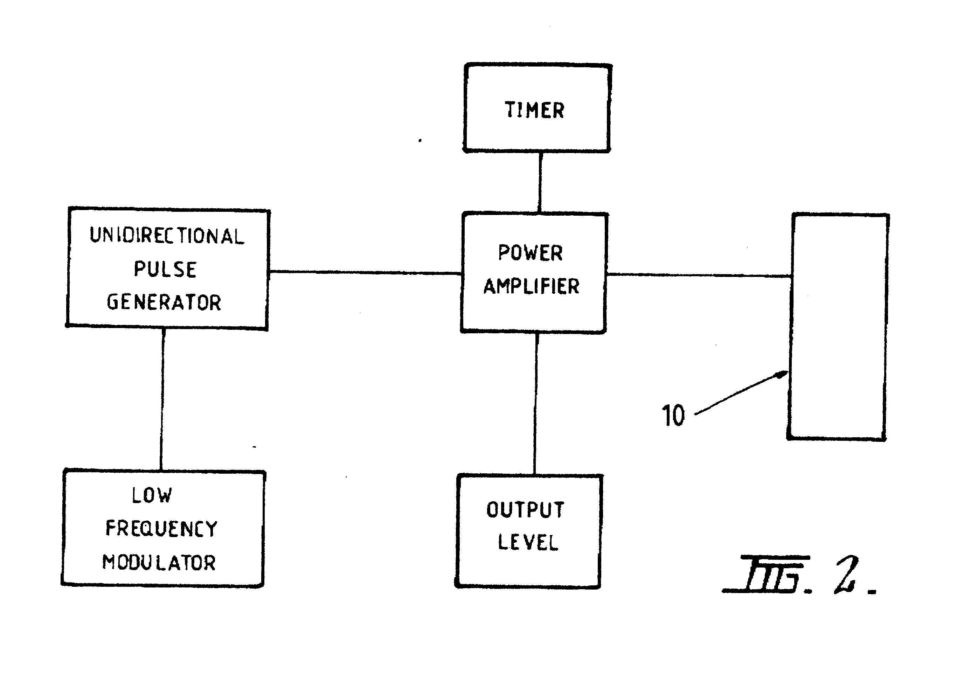

In order to provide the required current to the coil, I provide circuitry, which may be as illustrated in Fig. 2, and which can be readily connected to power available in a vehicle, such as to a cigarette lighter. The circuitry includes a pulse generator, which is a unidirectional pulse generator, which pulse generator is modulated by a low frequency modulator.

The low frequency modulator modulates the pulse generator on a cycle time of 20 milleseconds, during which time the pulse is on for 25% of the time and off for 75% of the time.

The pulse generator is adapted to provide a pulse train which has a pulse length of 20 milliseconds during 25% of which, or 5 milliseconds, the pulse is on and during the remainder the pulse is off and the low frequency modulator modulates the pulse generator so that the pulses are cyclicly generated or not generated with the cycles being 100 milliseconds on and 100 milliseconds off. Thus there are five pulses produced, a gap for 100 milliseconds, another five pulses and so on and, during a second, there is a composite repetition rate of when pulses are occurring of 5 Hz.

The output from the pulse generator passes to a power amplified which has associated therewith both a timer arrangement and an output level arrangement.

0209246

The output level is adjusted to normally provide approximately 50 gauss at the coils 11, 12 of the pad 10 which would give approximately 15 gauss where the field comes into contact with a user.

The timer is provided to restrict the amplification and transmission of pulses for half an hour in a four hour period, although the specific times can be varied.

The arrangement is such that, notwithstanding the fact that the ignition of the vehicle may be switched on and switched off, the timer retains a memory so that, after a full period of treatment, which can be broken, there can be no further pulses transmitted for the minimum of a three and a half hour period.

In use, if the device is to be used to prevent muscle stiffness and pain in a vehicle driver's back the pad of the invention is located against the rear of the seat of the vehicle and is held in position thereabouts by means of the straps or the like 16 which are tied about the seat back.

The pulse source is connected to a source of power, such as by a cigarette lighter socket, and the operation of the device commences and continues as previously described for an on period of half an hour and an off period of three and a half hours.

The driver, in his normal driving position, has his back in close proximity to the pad 10, which is comfortable because of the lambswool covering, and provides magnetic waves to the muscles of the driver at the selected, which is a desired, frequency and at a power which is considered to be suitable.

If the driver should be suffering from muscle pains in the thighs or calves, it is possible that the device be located on the seat of the vehicle and extend thereover so that the magnetic field can be brought into contact with those muscles.

Further, although I have described the device as being useful in a vehicle situation, it will be perceived that. for persons with muscle soreness, the device could be used as an underblanket on a bed or chair so that, during rest, magnetic treatment of the body muscles could be provided.

Whilst I have described one particular form of manufacture of the pad of the invention, it will be perceived that the coils could be located in a material other than a synthetic plastics foam and, also, that it would not be necessary that the outer cover be lambswool, although it would be desirable that the cover be of a material which is comfortable against a user and which can stand substantial wear.

Also, although not described, it may be preferred that the cover be able to be able to be replaceably removeable from the coil portion of the pad, so as to be able to be washed and replaced.

Specific intensity, frequency and treatment time have been mentioned, however, intensities of between 1 – 200 gauss, frequencies of between 1 – 200 Hz and up to two hours on and two hours off in all combinations of the above have proved to be of therapeutic value.

0209246

CLAIMS:

1.  A magnetic therapy device being a flexible pad (10) which is adapted to be in close contact with a user when in use, which pad includes at least one elongated magnetic coil (11, 12) which is adapted to be located adjacent the portion of the body of the user to be treated, a support (13) upon which this coil is located and a covering (15) for the coil.

2.  A magnetic therapy device as claimed in claim 1 wherein there are at least two magnetic coils (11, 12), adjacent coils being of opposite polarity.

3.  A magnetic therapy device as claimed in claim 1 or claim 2 wherein the coil(s) is/are sandwiched between two layers (13) of a material having no magnetic properties.

4.  A magnetic therapy device as claimed in claim 3 wherein the material of the sandwich (13) is a synthetic fibre material.

5.  A magnetic therapy device as claimed in claim 3 or claim 4 wherein the device has an outer cover (15).

6.  A magnetic therapy device as claimed in any preceding claim and adapted for use on the seat back of a vehicle including means (16) whereby the device can be connected to the seat back.

7.  A magnetic therapy device as claimed in any one of claims 1 to 5 and adapted to be wrapped about portion of a persons body and having means whereby it can be retained thereabout.

8.  A magnetic therapy system including a magnetic therapy device as claimed in any preceding claim and including means to produce a magnetic field in the coil(s) of the device which means includes a pulse generator and a power amplifier.

9.  A magnetic therapy system as claimed in claim 8 wherein the means to produce a magnetic field in the coil(s) includes modulating means to cyclicly control the output of the pulse generator so that the pulses are produced in bursts.

10.  A magnetic therapy system as claimed in claim 8 or claim 9 wherein the means to produce a magnetic field in the coil(s) includes a timer to control the maximum period during which pulses can be sent from the power amplifier to the coil(s) in a specific time.

11.  A magnetic therapy device substantially as hereinbefore described with reference to the accompanying drawings.

12.  A magnetic therapy system substantially as hereinbefore described with reference to the accompanying drawings.

_Fig. 1._

0209246

FIG. 2.

TIMER

POWER AMPLIFIER

OUTPUT LEVEL

UNIDIRECTIONAL PULSE GENERATOR

LOW FREQUENCY MODULATOR

10

# EUROPEAN SEARCH REPORT

European Patent Office

## DOCUMENTS CONSIDERED TO BE RELEVANT

EP 86304529.0

| Category | Citation of document with indication, where appropriate, of relevant passages | Relevant to claim | CLASSIFICATION OF THE APPLICATION (Int. Cl.4) |
|---|---|---|---|
| X | DE - A1 - 3 331 976 (MÜNCHINGER)<br>* Abstract; claims 1,7,8; fig. 2,3 * | 1,3,5,8 | A 61 N 1/42<br>B 60 N 1/00 |
| X | DE - A - 2 116 869 (KRAUS)<br>* Claims 1,9-11; fig. 1 * | 1,3,8 | |
| X | EP - A1 - 0 126 805 (ELECTRO-BIOLOGY)<br>* Page 8, line 19 - page 10, line 21; fig. 1-5b,7 * | 1,3,5,7-10 | |
| X | EP - A1 - 0 104 793 (ELECTRO-BIOLOGY)<br>* Page 6, line 2 - page 10, line 18; page 11, lines 5-15; fig. 1,3 * | 1-3,5,7-10 | |
| X | EP - A1 - 0 042 889 (ELECTRO-BIOLOGY)<br>* Abstract; fig. 1-5b,7 * | 1-3,5,7-10 | TECHNICAL FIELDS SEARCHED (Int. Cl.4)<br><br>A 61 N<br>B 60 N<br>A 47 C |
| A | WO - A1 - 85/1 881 (NIHONDENJI-HACHIRYOKIKENYUSHO)<br>* Abstract; fig. 1,5,6 * | 1,7-9 | |
| A | DE - A1 - 3 128 263 (BUSCHKY)<br>* Abstract; claims 1-6 * | 1,8,9 | |
| A | GB - A - 1 323 222 (FYNN)<br>* Claims 1,7-9; fig. 1-3 * | 1,6 | |

The present search report has been drawn up for all claims

| Place of search | Date of completion of the search | Examiner |
|---|---|---|
| VIENNA | 24-09-1986 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

**European Patent Office**

# EUROPEAN SEARCH REPORT

-2-
EP 86304529.0

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl.4) |
| A | AUSZÜGE AUS DEN GEBRAUCHSMUSTERN, vol. 21, no. 14, Munich, April 5, 1984 page 1131 DE-U-8 407 018 | 1,6 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 24-09-1986 | NEGWER |